# EUROPEAN PATENT SPECIFICATION

(11) **EP 1 836 975 B1**
(45) Date of publication and mention of the grant of the patent: **30.09.2009**
(21) Application number: 07250750.2
(22) Date of filing: 22.02.2007
(51) Int. Cl.: A61B 17/15, A61B 19/00

(54) **A surgical instrument for locating a cutting plane on a bone**
Chirurgisches Instrument zur Lokalisierung einer Schnittebene auf einem Knochen
Instrument chirurgical pour localiser le plan de coupe d'un os

(30) Priority: 09.03.2006 GB 0604757
(43) Date of publication of application: 26.09.2007
(73) Proprietor: DePuy International Limited, St. Anthony's Road Beeston, Leeds LS11 8DT (GB)
(72) Inventor: Cawthan, Simon, Shipley BD18 4QP (GB); Dower, Liam, Shelley, Huddersfield HD8 8JX (GB)
(74) Representative: Belcher, Simon James

(56) References cited:
- EP-A- 1 559 375
- EP-A- 1 574 171
- WO-A-98/32384
- WO-A-20/04075767

## Description

This invention relates to an instrument for locating a cutting plane on a bone in an orthopaedic surgical procedure.

Many orthopaedic surgical procedures, especially for the implantation of a joint prosthesis, involve cutting (including drilling) the bone in a preparatory step. It is important in such procedures to locate the cutting plane accurately to ensure that implanted components are positioned appropriately, and to ensure that damage to the bone is minimised.

To enable such suitably oriented cuts to be made, surgeons currently use cutting guides to align a cutting tool (such as a saw or a drill) correctly. Such guides are commonly used with alignment rods which can be intramedullary rods or extramedullary rods. The position and orientation of a cutting guide can be adjusted relative to an alignment rod on which it is mounted.

A disadvantage of the use of an alignment rod is that it requires an initial step of positioning the rod accurately relative to the bone on which the procedure is to be performed. This requirement can be obviated if a cutting block is positioned by freehand adjustment, or if the position of the cutting block is monitored using tracking apparatus, for example using magnetic or optical tracking components.

WO-98/32384 discloses an instrument for use in shaping a femur prior to implantation of a knee joint prosthesis. The instrument comprises parallel opposed arms having pins which can engage opposite medially and laterally facing surfaces of the femur, connected by a transverse cross-piece. The cross-piece carries a stylus which can slide in the cross-piece.

The present invention provides an instrument for locating a cutting plane on a bone in an orthopaedic procedure, which has a reference portion defining a reference plane and which presents three projections to contact the bone in a stable tripod arrangement, in which one of the projections can be moved relative to the other projections in a plane which is parallel to the reference plane.

Accordingly, in one aspect, the invention provides an instrument as defined in claim 1.

The instrument of the invention provides the advantage that it can position a cutting guide (provided by the reference portion) stably relative to a bone, quickly and easily. This advantage arises from the tripod arrangement of the projections which can provide three points of contact with the bone, to provide the required stability. It has been found that the stability provided by this arrangement is sufficient to enable the reference portion of the instrument to be used directly, without separate attachment (direct or indirect) of the reference portion to the bone, to guide a cutting tool in a cutting step of the procedure. Alternatively, the reference portion may be attached to the bone, for example by means of pins or screws, to provide a secure guide for the cutting tool.

The tripod arrangement of the projections can be achieved by providing one projection on the body portion and two projections on the reference portion. A single projection on the body portion allows movement of the reference portion in multiple degrees of freedom when the body portion projection is placed against the bone. The triple point contact is achieved by relative sliding movement between the two projections of the reference portion and the projection on the body portion.

Preferably, two projections are provided on the body portion, and a single projection is provided on the reference portion. This can have the advantage of providing greater stability of the body portion while the reference portion is positioned. In this preferred arrangement, when the two projections of the body portion are in contact with the bone, the reference portion is limited to a single axis of rotation (around the axis which is defined by the tips of the projections). In this way, the body portion projections may be used to locate the position of the instrument along the bone, and the angle of between the reference plane and a first plane which contains the bone axis. These parameters are then fixed if the projections on the body portion are not moved relative to the bone, while the other projection is positioned so as to fix the angle between the reference plane and a second plane containing the bone axis which is perpendicular to the said first plane. For example, the body portion projections may be positioned on the bone so as to fix the inclination of the reference plane around the medial/lateral axis, with the inclination of the reference plane around the anterior/posterior axis being determined by relative movement between the projection on the reference portion and the projections on the body portion.

When two projections are provided on the body portion, these projections are placed on to the bone at or at least close to the required position of the reference plane along the bone. The position of the body portion can then be adjusted finely by tilting it so that just one of the projections on the body portion is in contact with the bone, rotating the instrument about that projection, and then tilting the instrument back towards the bone so that both of the projections are once again in contact with the bone. Once the body portion has been positioned in this way, the instrument can be tilted about an axis defined by the ends of the projections on the body portion to vary the orientation of the reference plane. The reference portion of the instrument can then be fixed in this position by relative movement between the projection on the reference portion and the projections on the body portion, until all of the projections are in contact with the bone.

When there are two or more projections on the body portion or the reference portion, those projections can be capable of relative sliding movement.

The or each projection on the body portion might be capable of sliding relative to the body portion. Preferably, the or each projection on the body portion is fixed against sliding relative to the body portion.

The or each projection on the reference portion might be capable of sliding relative to the reference portion. Preferably, the or each projection on the reference portion is fixed against sliding relative to the reference portion.

When two or more projections are provided on the body portion or the reference portion and are capable of sliding relative to the body portion or reference portion, it will often be preferred that relative sliding between the projections can be restricted.

It will generally be preferred that the or each projection on the body portion is connected rigidly to the body portion, for example as a result of being formed integrally with the body portion (for example by a moulding process when this is appropriate in view of the material that is used) or being formed separately and then bonded to the body portion. Similarly, it will generally be preferred that the or each projection on the reference portion is connected rigidly to the reference portion, for example as a result of being formed integrally with the reference portion (for example by a moulding process when this is appropriate in view of the material that is used) or being formed separately and then bonded to the reference portion. These arrangements can simplify manufacture. They can reduce the number of separable parts of the instrument which can simplify cleaning.

The projections are shaped such they provide point contact with the bone, to account for the uneven bone surface. This can help to locate the instrument stably against the bone surface. It will generally be preferred that the projections have a sharp tip or a rounded (convex) tip. The surface area and shape of the tip will depend on the surface of the bone which the projections will contact, and the degree of required stability. The projections can be tapered along at least part of their length towards the tip. The projections can be in the form of pins with an approximately constant cross-section along most or all of their length. The projections are preferably oriented parallel to one other. Alternatively the pins may be oriented toward or away from each other to alter the size of the support base. The pins may be separate members or integrally formed with the body portion or the reference portion.

Preferably, the reference portion can slide relative to the body portion. This enables relative movement between the or each projection on the reference portion and the or each projection on the body portion. Relative linear movement between the reference portion and the body portion may be achieved by any suitable means. Preferably, one of the reference portion and the body portion is provided with a rib, and the other is provided with a corresponding groove in which the rib is a sliding fit. Preferably, the rib and the groove are configured so that the rib is retained within the groove with movement of the reference portion relative to the body portion restricted to movement along the groove. Preferably, the rib and groove are a dovetail arrangement. It has been found that a simple rib and groove arrangement is advantageous as it allows the reference portion to be easily removed from the body portion for cleaning.

Preferably, the instrument includes a locking mechanism by which relative movement between the projections on the reference portion and the projections on the body portion can be prevented. This enables the orientation of the cutting plane to be fixed in position once it has been determined. The locking mechanism may be a clamp, or a catch or ratchet mechanism, or any other suitable locking mechanism. Preferably the locking mechanism includes a threaded fixing member such as a grub screw, which passes through a corresponding threaded aperture in the body portion or the reference portion, to contact and clamp against a respective projection.

The reference plane defined by the reference portion may used to guide a tool by which a bone is resected, especially to form a planar resection surface on the bone. The reference plane which is defined by the reference portion can used to guide a tool by which one or more bores is formed in the bone, such as a drill. The reference portion may include a generally flat upper surface which defines a reference plane for a cutting blade. Alternatively, the reference portion may be provided with at least one guide hole which defines the reference plane for a drilling tool (when there is just one hole, the reference plane will be any plane which contains the hole). The reference portion may be provided with interchangeable components such that the reference plane defined by the reference portion may be adapted to suit the required application. In an embodiment of the invention the reference portion may include a substantially flat upper surface defining a first reference plane, and attachment points for attachment of a guide hole portion to define a second reference plane. Alternatively, or additionally, the reference may include a guide slot to receive, and define the cutting plane for, a cutting blade.

The instrument may include a device which enables the position of the reference portion to be monitored. The monitoring device might be part of the reference portion or the body portion. The monitoring device might be a separate device which can be connected directly or indirectly to the reference portion. The monitoring device can enable the position of the reference portion to be tracked using techniques such as magnetic tracking or optical tracking. The tracking device enables the position of the reference portion and hence the reference plane, to be more accurately determined, especially with reference to images of the patient's bone obtained in a pre-operative planning stage.

It is particularly preferred that the monitoring device is provided as a separate device which can be connected to the reference portion. Preferably, the monitoring device includes a monitoring device reference surface, which can be placed in contact with the reference plane on the reference portion to register the monitoring device with the reference portion.

Preferably, the instrument includes a handle which is connected to the body portion. The handle assists manipulation and orientation of the instrument. The handle can extend from the base of the body portion. The handle can be detachable from the body portion. Preferably the handle and the body portion are rigidly fastened to one another, for example as a result of being formed integrally.

The instrument can be made from materials which are commonly used in the manufacture of surgical instruments. Polymeric materials can be preferred for their light weight and the possibility of manufacture using moulding techniques. Examples of polymeric materials might include for example polyolefins, polyesters, polyamides, polycarbonates and similar materials. Metallic materials can be preferred for their resistance to wear. Components can be made from metallic materials by techniques such as casting and machining. Suitable metallic materials include certain stainless steels, as used commonly in the manufacture of surgical instruments.

The instrument of the invention can be used in procedures for the replacement of orthopaedic joints such as ankle, knee, hip, elbow and shoulder. It is particularly suitable for use in procedures in which a bone has to be resected on a plane, for example in preparing the tibia to receive the tibial component of a knee joint prosthesis, or to prepare the humerus to prepare the humeral component of a shoulder joint prosthesis.

Embodiments of the invention will now be described by way of example with reference to the accompanying drawings, in which:
Figure 1 is an isometric view of the instrument of the invention with the reference and body portions disassembled.
Figure 2 is an isometric view of the instrument of the invention with the reference and body portions assembled, and including a plane finder instrument.
Figure 3 is an enlarged isometric view from above of the bone facing ends of reference and body portions of the instrument.
Figure 4 is a side view of the instrument of the invention in contact with a bone model.

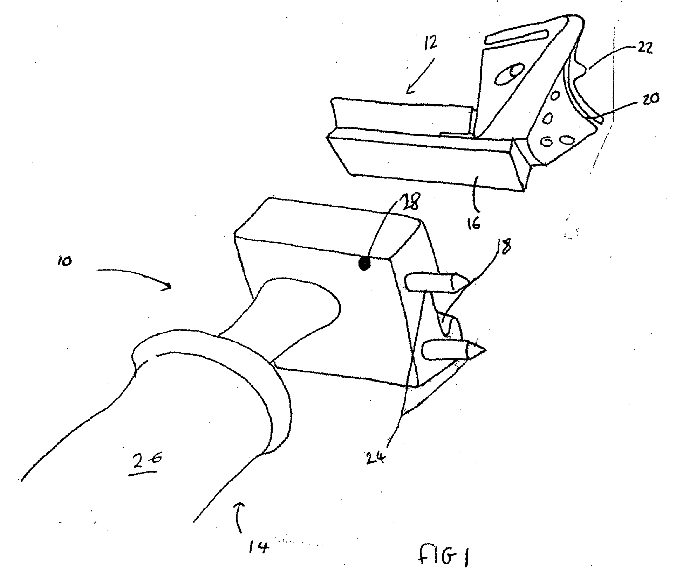

Referring to the drawings, Figures 1 to 3 show an instrument 10 for locating a cutting plane on a bone in an orthopaedic surgical procedure, such as to locate the cutting plane on a tibia in a procedure to implant a knee joint prosthesis. The instrument 10 comprises a reference portion 12 and a body portion 14. The reference portion 12 has a rib 16 formed on its lower surface with a cross-section such that it is wider at a point spaced from the reference portion than close to the reference portion (a "dovetail" cross-section). The body portion 14 has a groove 18 formed in its upper surface with a cross-section that is wider within the groove than at its opening. The rib on the reference portion is a sliding fit in the groove in the body portion.

The reference portion 12 has an upstand 20 at one end, which defines a slot in which a cutting tool such as a saw blade can be slid. The slot defines a reference plane for a cutting step in the surgical procedure. The reference plane is parallel to the direction defined by the rib on the reference portion. The reference portion has a projection 22 in the form of a pin extending from it, in a direction which is towards the bone which is to be resected when the instrument is in use. The pin is tapered to a point at its tip. The reference portion might be used to define a cutting plane for other cutting tools such as, for example, a drill or a burr.

The body portion has two pins 24 extending from it, in a direction which is towards the bone which is to be resected when the instrument is in use. Each of the pins is tapered to a point at its tip. The body portion also has a handle 26 by which it can be manipulated.

A threaded bore is provided in the body portion 10 which communicates with the groove 18 in the body portion. A screw 28 is provided in the threaded bore which, when screwed into the bore, engages the rib 16 on the reference portion 12, to lock the reference portion against sliding relative to the body portion 10.

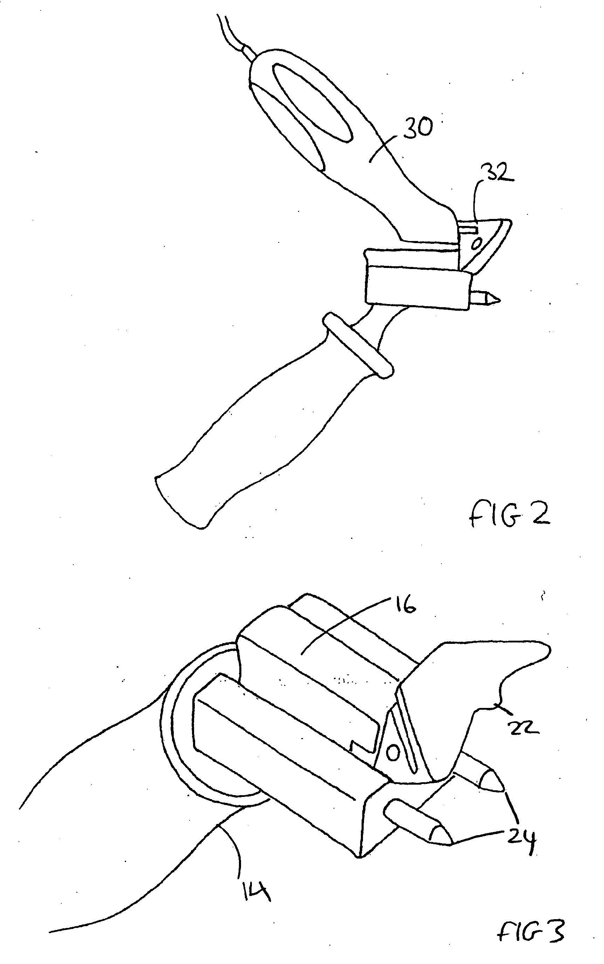

Figure 2 shows the instrument shown in Figure 1 with a plane finder instrument 30. The plane finder instrument includes a plate 32 which can be received snugly in the slot in the upstand 20 on the reference portion. The plane finder instrument can provide data as to the position (including orientation) of the plate 32. The position of the plane finder can be monitored remotely, for example using known magnetic or opto-electronic tracking apparatus. The position of the reference portion can be tracked in this way, and compared with position information derived from pre-operative planning stage of the surgical procedure. In other circumstances, a surgeon might prefer to position the reference plane relative to the bone by visual inspection.

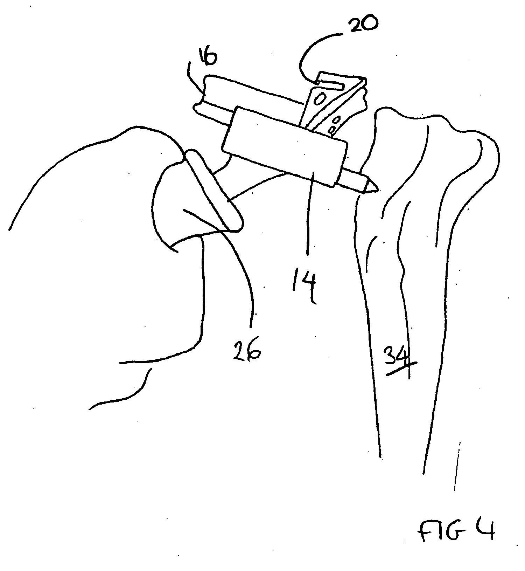

Figure 4 shows the instrument in position against a tibia model 34. The pins 24 are in contact with the anterior cortex of the tibia. The position of the instrument along the axis of the tibia can be adjusted by tilting it so that just one of the projections on the body portion is in contact with the bone, rotating the instrument about that projection, and then tilting the instrument back towards the bone so that both of the projections are once again in contact with the bone. These movements can also be used to adjust the orientation of the instrument about the anterior-posterior axis, which enables the varus-valgus orientation of the cutting plane to be set.

Once the body portion 10 has been positioned in this way, the instrument can be tilted about an axis defined by the ends of the projections on the body portion to vary the orientation of the reference plane about the medial-lateral axis. This enables the anterior-posterior slope of the cutting plane to be set.

The reference portion 12 of the instrument can then be fixed in this position by sliding the reference portion relative to the body portion the projection 22 on the reference portion, as well as the projections on the body portion, is in contact with the bone.

The reference portion can be used to guide the blade of a saw in a cutting step of the surgical procedure by receiving the blade in the slot in the upstand 20. It can be preferred for some procedures to fix the reference portion to the bone, for example by means of fixation pins, in order to provide additional stability during the cutting step.

## Claims

1. An instrument (10) for locating a cutting plane on a bone in an orthopaedic surgical procedure, comprising:
a reference portion (12) for placing against a surface of the bone, having a cutting guide feature which can guide a tool which is used to cut the bone in the surgical procedure, the cutting guide feature defining a reference plane,
a body portion (14), and
at least three projections (22, 24) which can contact the bone,
**characterised in that** the projections are adapted to contact the surface of the bone, against which the reference portion is placed, in a stable tripod arrangement, in which at least one of the projections (22) is provided on the reference portion, and the or each other projection (24) is provided on the body portion, with the projections on the reference portion being capable of linear movement relative to the projections on the body portion, towards and away from the surface of the bone in a plane which is parallel to the reference plane.

2. The instrument of claim 1, in which the body portion (14) comprises at least two projections (23).

3. The instrument of claims 1 or claim 2, in which each of the projections (22, 24) provides point contact with the bone.

4. The instrument of any preceding claim, in which the reference portion (12), with its projection(s) (22), is slideably mounted on the body portion (14).

5. The instrument of claim 4, in which one of the reference portion (12) and the body portion (14) is provided with a track (16), and the other is provided with a corresponding groove (18) for receiving the track, and in which the track can slide in the groove.

6. The instrument of claim 5, in which the track (16) and groove (18) are a dovetail arrangement.

7. The instrument of claim 4, which includes a lock (28) for locking the reference portion (22) against sliding movement relative to the body portion (24).

8. The instrument of any preceding claim, in which there are two projections (24) on the body portion (14) which are fixed against movement relative to one another.

9. The instrument of any preceding claim, in which the reference portion (12) includes a generally flat upper surface which defines the reference plane.

10. The instrument of any of claims 1 to 8, in which the reference portion (12) includes at least one drill guide hole which defines the reference plane.

11. The instrument of any preceding claim, further comprising a monitoring device (30) for monitoring the position of the reference portion (12).

12. The instrument of claim 11, in which the monitoring device (30) can be fastened to the reference portion (12).

13. The instrument of claim 11, in which the monitoring device (30) is housed within the body portion (14).

14. The instrument of any preceding claim, in which the reference portion (12) is provided with attachment features to enable the reference portion to be attached to the bone.

15. The instrument of any preceding claim, which includes a handle (26) which is fastened to the body portion (14).

## Patentansprüche

1. Instrument (10) zum Lokalisieren einer Schneidebene auf einem Knochen bei einer orthopädischen chirurgischen Prozedur, das aufweist:
einen Referenzbereich (12), der gegen eine Oberfläche des Knochen gelegt wird, welcher ein Schneidführungsmerkmal aufweist, das ein Werkzeug führen kann, das verwendet wird, um den Knochen bei der chirurgischen Prozedur zu schneiden, wobei das Schneidführungsmerkmal eine Referenzebene definiert,
einen Körperbereich (14) und
wenigstens drei Vorsprünge (22, 24), die den Knochen berühren können,
**dadurch gekennzeichnet, dass** die Vorsprünge dazu ausgelegt sind, die Oberfläche des Knochens, gegen die der Referenzbereich gebracht wird, in einer stabilen dreibeinigen Anordnung zu berühren, bei der wenigstens einer der Vorsprünge (22) auf dem Referenzbereich vorgesehen ist und der oder jeder weitere Vorsprung (24) auf dem Körperbereich vorgesehen ist, wobei die Vorsprünge auf dem Referenzbereich zur linearen Bewegung in Bezug auf die Vorsprünge auf dem Körperbereich in der Lage sind, auf die Oberfläche des Knochens zu und von ihr weg, in einer Ebene, die parallel zu der Referenzebene liegt.

2. Instrument nach Anspruch 1, bei dem der Körperbereich (14) wenigstens zwei Vorsprünge (23) aufweist.

3. Instrument nach Anspruch 1 oder Anspruch 2, bei dem jeder der Vorsprünge (22, 24) einen Punktkontakt mit dem Knochen bildet.

4. Instrument nach einem der vorangehenden Ansprüche, bei dem der Referenzbereich (12), mit seinem Vorsprung / seinen Vorsprüngen (22) verschieblich auf dem Körperbereich (14) angeordnet ist.

5. Instrument nach Anspruch 4, bei dem entweder der Referenzbereich (12) oder der Körperbereich (14) mit einer Schiene (16) versehen ist und der andere mit einer entsprechenden Nut (18) zum Aufnehmen der Schiene versehen ist und bei dem die Schiene in der Nut gleiten kann.

6. Instrument nach Anspruch 5, bei dem die Schiene (16) und die Nut (18) eine Schwalbenschwanzanordnung haben.

7. Instrument nach Anspruch 4, das eine Sperre (28) zum Verriegeln des Referenzbereiches (22) gegen die Verschiebebewegung in Bezug auf den Körperbereich (24) umfasst.

8. Instrument nach einem der vorangehenden Ansprüche, bei dem es zwei Vorsprünge (24) auf dem Körperbereich (14) gibt, die gegen die Bewegung in Bezug aufeinander festgehalten sind.

9. Instrument nach einem der vorangehenden Ansprüche, bei dem der Referenzbereich (12) eine im Allgemeinen flachere obere Fläche umfasst, welche die Referenzebene definiert.

10. Instrument nach einem der Ansprüche 1 bis 8, bei dem der Referenzbereich (12) wenigstens ein Bohrführungsloch umfasst, welches die Referenzebene definiert.

11. Instrument nach einem der vorangehenden Ansprüche, weiter mit einer Überwachungsvorrichtung (30) zum Überwachen der Position des Referenzbereiches (12).

12. Instrument nach Anspruch 11, bei dem die Überwachungsvorrichtung (30) an dem Referenzbereich (12) befestigt werden kann.

13. Instrument nach Anspruch 11, bei dem die Überwachungsvorrichtung (30) innerhalb des Körperbereiches (14) untergebracht ist.

14. Instrument nach einem der vorangehenden Ansprüche, bei dem der Referenzbereich (12) mit Befestigungsmerkmalen versehen ist, um zu ermöglichen, dass der Referenzbereich an dem Knochen befestigt wird.

15. Instrument nach einem der vorangehenden Ansprüche, das einen Griff (26) umfasst, der an dem Körperbereich (15) befestigt ist.

## Revendications

1. Instrument (10) destiné à localiser un plan de coupe sur un os au cours d'une procédure chirurgicale orthopédique, qui comprend :
- une partie de référence (12) destinée à être positionnée contre une surface de l'os, qui a un élément de guide de coupe qui peut guider un outil utilisé pour couper l'os durant la procédure chirurgicale, l'élément de guide de coupe définissant un plan de référence,
- une partie de corps (14), et
- au moins trois projections (22, 24) qui peuvent contacter l'os,
**caractérisé en ce que** les projections sont adaptées pour contacter la surface de l'os, contre laquelle la partie de référence est placée, en une configuration tripode stable, au moins une des projections (22) étant fournie sur la partie de référence, et l'autre ou les autres projection(s) (24) sont fournies sur la partie de corps, les projections sur la partie de référence étant capables de mouvement linéaire par rapport aux projections sur la partie de corps, en direction et à l'opposé de la surface de l'os dans un plan qui est parallèle au plan de référence.

2. Instrument selon la revendication 1, dans lequel la partie de corps (14) comprend au moins deux projections (23).

3. Instrument selon la revendication 1 ou la revendication 2, dans lequel chacune des projections (22, 24) fournit un point de contact avec l'os.

4. Instrument selon l'une quelconque des revendications précédentes, dans lequel la partie de référence (12), avec sa (ses) projection (s) (22), est montée coulissante sur la partie de corps (14).

5. Instrument selon la revendication 4, dans lequel l'une des parties de référence (12) et de la partie de corps (14) est dotée d'un rail (16), et l'autre est dotée d'un sillon correspondant (18) pour recevoir le rail, et dans lequel le rail peut coulisser dans le sillon.

6. Instrument selon la revendication 5, dans lequel le rail (16) et le sillon (18) sont assemblés en queue d'aronde.

7. Instrument selon la revendication 4, qui comprend un verrou (28) destiné à empêcher un mouvement de coulissement de la partie de référence (22) par rapport à la partie de corps (24).

8. Instrument selon l'une quelconque des revendications précédentes, dans lequel deux projections (24) sur la partie de corps (14) ne peuvent pas se déplacer l'une par rapport à l'autre.

9. Instrument selon l'une quelconque des revendications précédentes, dans lequel la partie de référence (12) comprend une surface supérieure généralement plate qui définit le plan de référence.

10. Instrument selon l'une quelconque des revendications 1 à 8, dans lequel la partie de référence (12) comprend au moins un trou pour guide-foret qui définit le plan de référence.

11. Instrument selon l'une quelconque des revendications précédentes, qui comprend en outre un dispositif de surveillance (30) destiné à surveiller la position de la partie de référence (12).

12. Instrument selon la revendication 11, dans lequel le dispositif de surveillance (30) peut être fixé à la partie de référence (12).

13. Instrument selon la revendication 11, dans lequel le dispositif de surveillance (30) est logé à l'intérieur de la partie de corps (14).

14. Instrument selon l'une quelconque des revendications précédentes, dans lequel la partie de référence (12) est dotée d'éléments de fixation destinés à permettre la fixation de la partie de référence à l'os.

15. Instrument selon l'une quelconque des revendications précédentes, qui comprend une poignée (26) fixée à la partie de corps (14).
